# EUROPÄISCHE PATENTSCHRIFT

(11) **EP 3 984 566 B1**
(45) Veröffentlichungstag und Bekanntmachung des Hinweises auf die Patenterteilung: **02.08.2023**
(21) Anmeldenummer: 21203449.0
(22) Anmeldetag: 19.10.2021
(51) Int. Cl.: A61L 9/20

(54) **HYGIENELEUCHTE**
HYGIENE LIGHT
DISPOSITIF D'ÉCLAIRAGE D'HYGIÈNE

(30) Priorität: 19.10.2020 DE 202020105951 U
(43) Veröffentlichungstag der Anmeldung: 20.04.2022
(73) Patentinhaber: Ruco-Licht GmbH, 86179 Augsburg (DE)
(72) Erfinder: WEINDL, Markus, 86343 Königsbrunn (DE)
(74) Vertreter: Charrier Rapp & Liebau

(56) Entgegenhaltungen:
- KR-A- 20140 136 779
- KR-A- 20190 110 972
- KR-Y1- 200 450 704
- US-A1- 2017 321 877
- US-A1- 2020 278 096

## Beschreibung

Die Erfindung betrifft eine Hygieneleuchte nach dem Oberbegriff des Anspruchs 1.

Eine derartige Hygieneleuchte ist aus der KR 200 450 704 Y1 bekannt. Eine dort offenbarte Hygieneleuchte enthält einen Korpus, in dem eine Lichteinheit sowie ein Desinfektionsmodul angeordnet sind. Das Desinfektionsmodul umfasst ein Gehäuse mit einem Einlass, einem Auslass und einem sich dazwischen erstreckenden Strömungskanal. Das Desinfektionsmodul umfasst auch eine im Strömungskanal angeordnete UV-Leuchteinheit. Der Korpus der bekannten Hygieneleuchte enthält einen zu dessen Außenseite hin offenen Einbauschacht, in welchem das Desinfektionsmodul mit seinem Gehäuse als gesamte Einheit demontierbar aufgenommen ist. Die Hygieneleuchte enthält auch ein Gebläse, das an dem Korpus der Hygieneleuchte befestigt ist.

Aus dem Stand der Technik sind mit der KR 2019 0110 972 A und der KR 2014 0136 779 A ferner weitere Hygieneleuchten bekannt, die jeweils eine Beleuchtungsfunktion und eine keimabtötende Funktion durch Einsatz von UV-Licht besitzen.

Aus der US 2020 027 809 6 A1 ist außerdem ein Laternenpfahl bekannt, der durch Einbau unterschiedlicher Module wie z.B. einem WLAN-Modul oder einem Kamera-Modul anwendungsspezifisch erweiterbar ist.

Um eine gleichbleibende Reinigungsleistung zu erzielen, müssen Hygieneleuchten in regelmäßigen Abständen gewartet werden. Dabei müssen der Strömungskanal und vor allem die UV-Leuchteinheit von im Laufe der Zeit sich mit der Luft eingetragenen und darin abgesetzten Schmutzpartikeln befreit werden. Bei den im Stand der Technik bekannten Hygieneleuchten muss hierzu die gesamte Hygieneleuchte abmontiert und der Strömungskanal freigelegt werden. Dies ist zeit- und ressourcenintensiv.

Vor diesem Hintergrund ist es Aufgabe der Erfindung, eine Hygieneleuchte mit geringerem Wartungsaufwand zur Verfügung zu stellen.

Diese Aufgabe wird durch eine Hygieneleuchte mit den Merkmalen nach Anspruch 1 gelöst.

Eine erfindungsgemäße Hygieneleuchte besitzt einen Korpus, in welchem eine Lichteinheit und ein Desinfektionsmodul angeordnet sind. Das Desinfektionsmodul umfasst dabei ein eigenes Gehäuse mit einem Einlass, einem Auslass und einem sich dazwischen erstreckenden Strömungskanal, wobei in dem Korpus ein zu dessen Außenseite hin offener Einbauschacht angeordnet ist, in welchem das Desinfektionsmodul als gesamte Einheit demontierbar aufgenommen ist. Die Hygieneleuchte zeichnet sich dadurch aus, dass in dem Desinfektionsmodul am Einlass und/oder am Auslass des Gehäuses ein als Axiallüfter ausgebildetes Gebläse senkrecht zu einer Strömungsrichtung angeordnet ist. Dadurch kann das Desinfektionsmodul zusammen mit dem Gebläse als Einheit zum Beispiel zu Wartungszwecken schnell und einfach aus der Hygieneleuchte entnommen werden. Eine Demontage der gesamten Hygieneleuchte entfällt. Außerdem kann das Desinfektionsmodul sofort durch ein gleichartiges und neuwertiges oder bereits gewartetes Desinfektionsmodul ersetzt werden. Die Wartung des entnommenen Desinfektionsmoduls muss daher nicht mehr vor Ort erfolgen. Für einen besonders flachen Aufbau der Hygieneleuchte ist das eine oder jedes Gebläse als Axiallüfter ausgebildet. Die Axiallüfter sind dabei senkrecht zu einer Strömungsrichtung im Strömungskanal des Desinfektionsmoduls (synonym: liegend, nicht stehend) angeordnet. Hierdurch können Abmessungen des bzw. der Axiallüfter und damit insbesondere die Förderleistung im Wesentlichen unabhängig von einer verfügbaren Höhe des Querschnitts des Strömungskanals gewählt werden.

Weitere Merkmale und vorteilhafte Abwandlungen der Erfindung ergeben sich aus den Unteransprüchen sowie der Figurenbeschreibung.

Der Einbauschacht ist vorteilhaft als einseitig offene Vertiefung ausgebildet, in der das Desinfektionsmodul vorzugsweise bündig abschließend aufgenommen sein kann. In der einseitigen Vertiefung kann das Desinfektionsmodul, insbesondere durch Einschieben längs einer Einschubachse, formschlüssig aufgenommen und sicher fixiert werden.

Vorteilhaft umfasst das Desinfektionsmodul eine elektrische und/oder mechanische Schnellkupplung, die mit einem komplementären Gegenstück im Einbauschacht gekoppelt werden kann. Über eine mechanische Schnellkupplung kann das Desinfektionsmodul einfach in dem Einbauschacht fixiert werden. Über eine elektrische Schnellkupplung können Steuerströme und/oder Leistungsströme übertragen werden. Besonders vorteilhaft ist es, wenn die Schnellkupplung eine mechanische sowie eine elektrische Schnittstelle vereint. Damit ist eine besonders schnelle Entnahme bzw. ein besonders schneller Austausch des Desinfektionsmoduls möglich. Die mechanische Schnellkupplung kann mit Rast- oder Klips-Elementen ausgebildet sein. Sie kann aber z.B. auch magnetisch ausgebildet sein.

Die Hygieneleuchte kann in einer vorteilhaften Ausführungsform eine Wartungsklappe aufweisen, welche an einer Oberseite des Desinfektionsmoduls angeordnet ist und über welche das Desinfektionsmodul bzw. dessen Strömungskanal unmittelbar ohne Ausbau des Desinfektionsmoduls zugänglich ist. Die Wartungsklappe kann zum Beispiel über ein Scharnier mit dem restlichen Gehäuse des Desinfektionsmoduls verbunden und über einen mechanischen Schnellverschluss gesichert sein. Wartungsklappe und Gehäuse bilden vorzugsweise dabei eine vollumfänglich dichtende Verbindung aus. Die Dichtwirkung erstreckt sich dabei auf das Abdichten von Gas als auch Luft, so das aus dem Strömungskanal (Einlass und Auslass ausgenommen) möglichst kein Licht (UV-Licht) oder Gas nach außen treten kann.

Das oder die Gebläse können saugend oder blasend arbeiten. Vorteilhaft ist es, wenn das Gebläse am Einlass blasend und das Gebläse am Auslass saugend arbeitet. Hierdurch können hohe gerichtete Volumenströme erreicht werden und/oder der Strömungskanal besonders lang ausgeformt werden. In einer bevorzugten Variante weist der Strömungskanal dabei eine Länge von wenigstens 1200 mm auf, besonders bevorzugt von mehr als 1500 mm. Durch eine entsprechende Länge des Strömungskanals können selbst bei hohen Volumendurchsätzen an Luft eine ausreichende Desinfektionswirkung erreicht werden.

Unter Axiallüfter sind Gebläse mit einer Rotationsachse zu verstehen, bei welchen die Luft parallel zur Rotationsachse gefördert wird. Beispielsweise handelt es sich bei einem Ventilator um ein Axiallüfter. Andere Gebläseformen, zum Beispiel Radialgebläse, sind möglich.

Die UV-Leuchteinheit kann als UV-Röhre ausgebildet sein. Es kann sich aber auch um einzelne oder mehrere LEDs mit UV-Strahlung handeln. Die UV-Lichteinheit erstreckt sich vorzugsweise über die gesamte Länge des Strömungskanals.

Zur Vermeidung des Austritts der UV-Strahlung nach außen sind im Strömungskanal nach dem Einlass sowie vor dem Auslass vorzugsweise jeweils eine Lichtfalle angeordnet. Unter Lichtfalle ist jede lichtdichte und luftoffene Vorrichtung zu verstehen, welche eine direkte Sichtverbindung hier zwischen dem Strömungskanal 54 sowie dem Einlass bzw. dem Auslass verhindert. Eine Lichtfalle kann beispielsweise als Labyrinth ausgestaltet sein.

Weiter vorteilhaft ist es, wenn im Strömungskanal wenigstens ein Reflektor bzw. ein Reflektorelement angeordnet oder ausgebildet ist. Ein Reflektor erfüllt den Zweck, von der UV-Leuchteinheit abgestrahltes Licht zurück zu Reflektoren, so dass möglichst viel der UV-Lichtleistung von der durch den Strömungskanal geleiteten Luft bzw. von in der Luft gelösten Schwebstoffen (Aerosolen) absorbiert wird und möglichst wenig Lichtleistung an den Seitenwänden des Strömungskanals in Wärme umgewandelt wird.

Besonders bevorzugt sind im Strömungskanal mehrere Reflektoren angeordnet, z.B. derart, dass das Licht der UV-Leuchteinheit permanent wieder zurück in die Mitte des Strömungskanals reflektiert wird. Die Reflektoren können insbesondere konkav, zum Beispiel als Hyberbol-Reflektor, ausgebildet sein. Dadurch kann der notwendige Energiebedarf zum Betreiben der UV-Leuchteinheit reduziert werden. Der oder die Reflektoren können sich dabei bevorzugt über die gesamte Länge des Strömungskanals erstrecken. Alternativ oder zusätzlich können die Seitenwände des Strömungskanals auch mit einer lichtreflektierenden Beschichtung wie beispielsweise einer metallischen Beschichtung beschichtet sein. Eine lichtreflektierende Beschichtung steht im Sinne dieser Schrift der Verwendung eines Reflektors gleich.

In einer Ausführungsform können die Lichteinheit und das Desinfektionsmoduls in Bezug auf ihre jeweilige Längsachse parallel zueinander ausgerichtet sein. Die Längsachse einer Baueinheit ist dabei i.A. in einem orthogonalen Koordinatensystem die Achse mit der längsten Erstreckung bzw. Abmessung. Die Längsachse des Desinfektionsmoduls wird dabei zum Beispiel durch den Strömungskanal bzw. eine Hauptströmungsrichtung im Strömungskanal festgelegt. Die Längsachse des Lichtmoduls wird im Falle einer Leuchtstoffröhre beispielsweise durch deren Längsachse, bei einer LED-Platine oder mehreren LED-Modulen durch deren Anordnung bestimmt.

Besonders zweckmäßig ist es, wenn die Lichteinheit dabei auf einer Seite der Hygieneleuchte und das Desinfektionsmoduls mit seinem Einlass und Auslass auf der gegenüberliegenden Seite der Hygieneleuchte angeordnet sind. Hierdurch kann die subjektive Geräuschkulisse, welche ein Nutzer der Hygieneleuchte wahrnimmt, reduziert werden.

In einer anderen zweckmäßigen Ausführungsform können die Lichteinheit und das Desinfektionsmodul auch senkrecht zueinander angeordnet sein. Vorteilhaft ist es auch hier, wenn der Einlass und der Auslass des Desinfektionsmoduls dabei von der Leuchteinheit wegweisen.

Die Hygieneleuchte kann beispielsweise als Pendelleuchte oder auch als Stehlampe ausgebildet sein.

Im Falle einer Stehlampe kann die Stehlampe einen Ständer und eine im Wesentlichen vertikal darauf aufbauende Säule umfassen, wobei der Einbauschacht für das Desinfektionsmodul im Ständer oder in der Säule, jedenfalls aber auf einer Außenseite, angeordnet sein kann. Die Positionierung in der Säule hat dabei den Vorteil, dass Luft aus unterschiedlichen Luftschichten umgewälzt werden kann und damit die Luftzirkulation verbessert.

Vorzugsweise ist der Einlass dabei (vertikal) unter dem Auslass angeordnet. Hierdurch kann Raumluft effektiver aufbereitet werden, da tiefer gelegene Luftschichten i.A. stärker mit Aerosolen belastet ist als darüber liegende Schichten.

Zur Ansteuerung von UV-Leuchteinheit, dem oder den Gebläsen sowie der Lichteinheit kann die Hygieneleuchte entsprechende Steuermodule für Licht, UV-Leuchteinheit bzw. Gebläse aufweisen.

Die Ansteuerungen können insbesondere auch in einem einzigen Steuermodul vereint sein. Das Steuermodul kann auch Software oder Programmcode zur Steuerung der einzelnen Komponenten (Gebläse, Lichteinheit, UV-Leuchteinheit) der Hygieneleuchte enthalten.

Das Steuermodul kann beispielsweise eine Schnittstelle zur Verbindung mit einem Schalter und/oder zu Anbindung an ein Gebäudemanagement-System aufweisen. Bei der Schnittstelle kann es sich insbesondere um eine Schnittstelle an ein Gebäude-Bus-System handeln. Über die Schnittstelle können beispielsweise die Funktionen des Desinfektionsmoduls ferngesteuert und/oder programmiert werden.

Vorteilhaft ist es, wenn das Steuermodul dabei die Leistung des bzw. der Gebläse und die Leistung der UV-Leuchteinheit unabhängig voneinander regelt. Dadurch ist es beispielsweise möglich, eine Desinfektionswirkung über einen nur passiven Luftaustausch im Strömungskanal ohne Zuschaltung der Gebläse zu realisieren (insbesondere unter Ausnutzung thermischer Effekte bei Positionierung der Hygieneleuchte in der Nähe einer Heizquelle oder Heizung) oder auch eine reine Luftumwälzung ohne desinfizierende Wirkung der UV-Leuchteinheit. Dadurch kann der Energiebedarf bedarfsgerecht gesteuert und der Gesamtenergieverbrauch gesenkt werden.

Die Hygieneleuchte kann vorteilhaft auch eine Zeitschaltuhr und/oder einen Bewegungsmelder zur automatischen An- und Abschaltung bzw. Steuerung des Lichtmoduls und/oder des Desinfektionsmoduls aufweisen.

Für eine einfache Wartung kann die Hygieneleuchte, insbesondere das Desinfektionsmodul, außerdem einen Sensor aufweisen. Dieser kann eingerichtet sein, einem Betreiber z.B. notwendige Wartungsarbeiten mittel- oder unmittelbar anzuzeigen. Beispielsweise kann es sich bei dem Sensor um eine Zähleinheit handeln, welche die Betriebsstunden der Hygieneleuchte bzw. des Desinfektionsmoduls zählt und bei Erreichen einer bestimmten Betriebszeit eine notwendige Wartung anzeigt. Es könnte sich aber auch um einen Volumenstrommesser handeln, welche zum Beispiel das Zusetzen eines im Strömungskanal befindlichen Filters anhand eines reduzierten Luftumsatzes bei ansonsten gleichen Leistungsparametern registriert und über eine Gebäudemanagement-System-Schnittstelle meldet. Der Sensor kann auch eingerichtet sein, den Zustand der Wartungsklappe (geöffnet vs. geschlossen) zu überwachen. Hierdurch kann das ungewollte Anschalten der UV-Leuchteinheit bzw. Austreten von UV-Licht aus dem Desinfektionsmodul bei geöffneter Wartungsklappe verhindert werden.

In einigen Ausführungsvarianten kann die Hygieneleuchte auch eine drahtgebundene oder drahtlose Kommunikationsschnittstelle aufweisen, über welche Zustandsinformationen zur Hygieneleuchte, insbesondere Zustandsinformationen zum Desinfektionsmodul oder der Leuchteinheit abgerufen oder weitergeleitet werden können. Über die Schnittstelle könnte auch die Leistung der Leuchteinheit und/oder des Desinfektionsmoduls geregelt werden. Die Kommunikationsschnittstelle kann z.B. eine Bluetooth-Schnittstelle sein. Durch eine entsprechende Schnittstelle kann die Hygieneleuchte App-fähig ausgestaltet werden, so dass die Hygieneleuchte zum Beispiel über ein Smartphone gesteuert werden kann.

Weitere Merkmale und vorteilhafte Ausprägungen der Erfindung ergeben sich aus der folgenden Beschreibung bevorzugter Ausführungsbeispiele und den Zeichnungen.

Dabei zeigen
- **Fig. 1**: eine Ausführungsform einer erfindungsgemäßen Hygieneleuchte als Stehlampe in einer perspektivischen Ansicht mit herausgenommenem Desinfektionsmodul,
- **Fig. 2**: eine Ausführungsform einer erfindungsgemäßen Hygieneleuchte als Pendelleuchte in einer 1. und einer 2. perspektivischen Ansichten mit herausgenommenem Desinfektionsmodul,
- **Fig. 3**: eine Ausführungsform einer erfindungsgemäßen Hygieneleuchte als Pendelleuchte in einer Draufsicht
- **Fig. 4**: die Hygieneleuchte aus Fig. 3 in einer Seitenansicht (Längsseite) mit herausgenommenem Desinfektionsmodul,

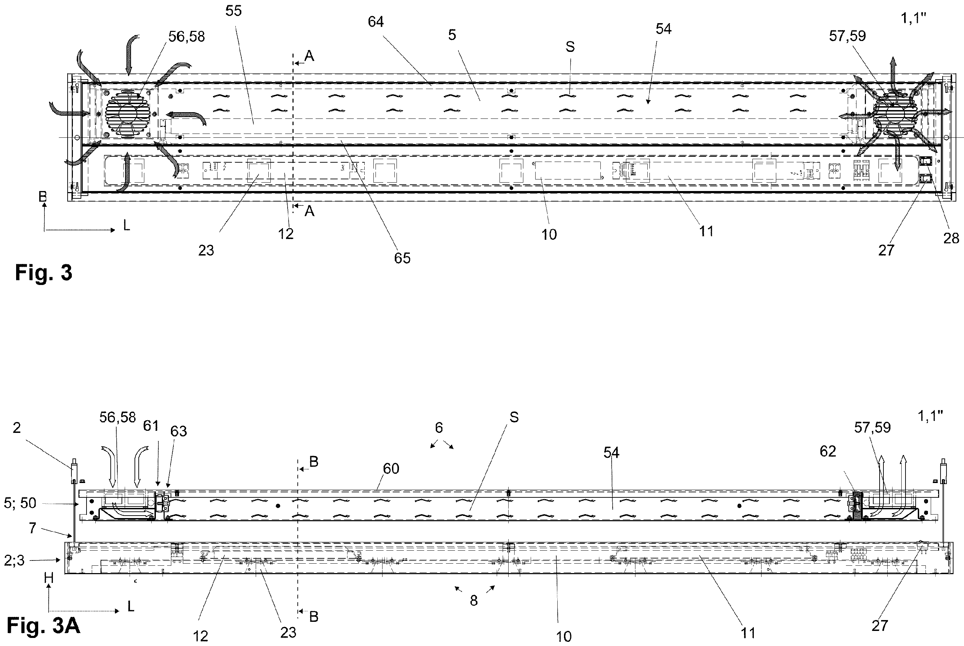

- **Fig. 3A**: eine Schnittansicht (zweite Seitenansicht) gemäß Schnitt A-A nach Fig. 3 (Breitseite) mit eingebautem Desinfektionsmodul

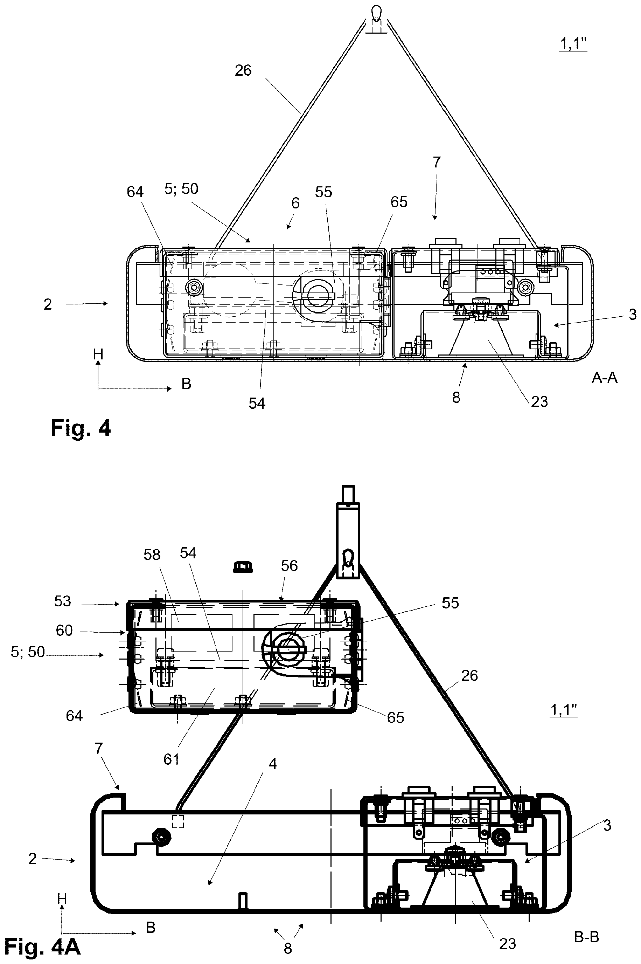

- **Fig. 4A**: eine Schnittansicht (Breitseite) gemäß Schnitt B-B nach Fig. 4 in einer 2. Seitenansicht mit exponiertem Desinfektionsmodul.

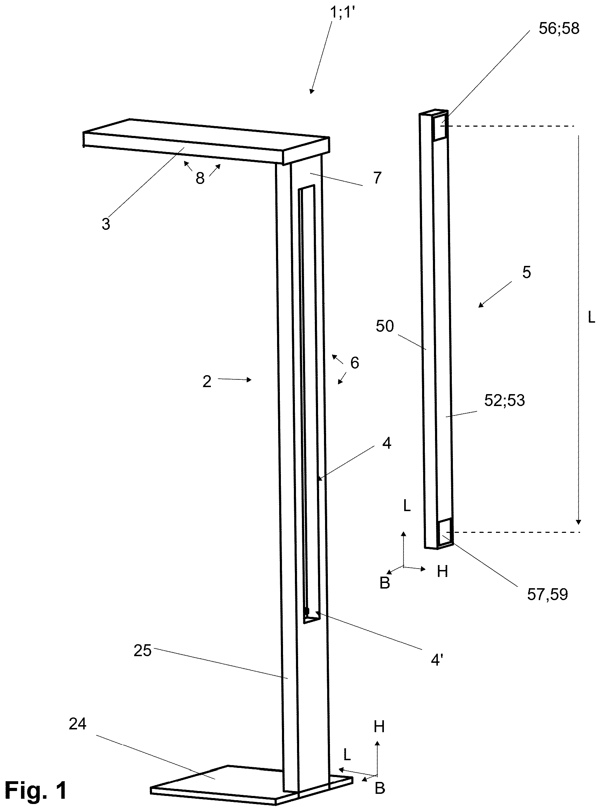

In Figur 1 ist eine Hygieneleuchte 1 in Form einer Stehlampe 1' gezeigt. Die Hygieneleuchte 1 besitzt einen Korpus 2, der aus einem Standfuß 24, einer sich in vertikaler Richtung (Hochachse H) erstreckenden Säule 25 mit einer Länge von etwa 220 cm sowie einem sich daran senkrecht, d.h. horizontal (Längsachse L), anschließenden Leuchteinheit 3 besteht. In der Leuchteinheit 3 sind hier nicht ersichtliche Leuchtmittel auf der Lichtseite 8 verbaut welche Licht nach unten abstrahlen. Alternativ oder zusätzlich könnten Leuchtmittel aber auch auf der gegenüberliegenden Seite des der Leuchteinheit 3 mit Strahlrichtung nach oben angeordnet sein.

In der Säule 2 ist ein Einbauschacht 4 in Form einer im Wesentlichen quaderförmigen zur Außenseite 7 des Korpus 2 hin offenen Vertiefung vorgesehen. In die Vertiefung ist ein Desinfektionsmodul 5 mit im Wesentlichen komplementären Abmessungen eingesetzt bzw. einsetzbar. Das Desinfektionsmodul 5 ist somit im eingebauten Zustand von fünf Seiten umschlossen und eine Oberseite 52 des Desinfektionsmoduls 5 schließt den Einbauschacht 4 im Wesentlichen bündig ab.

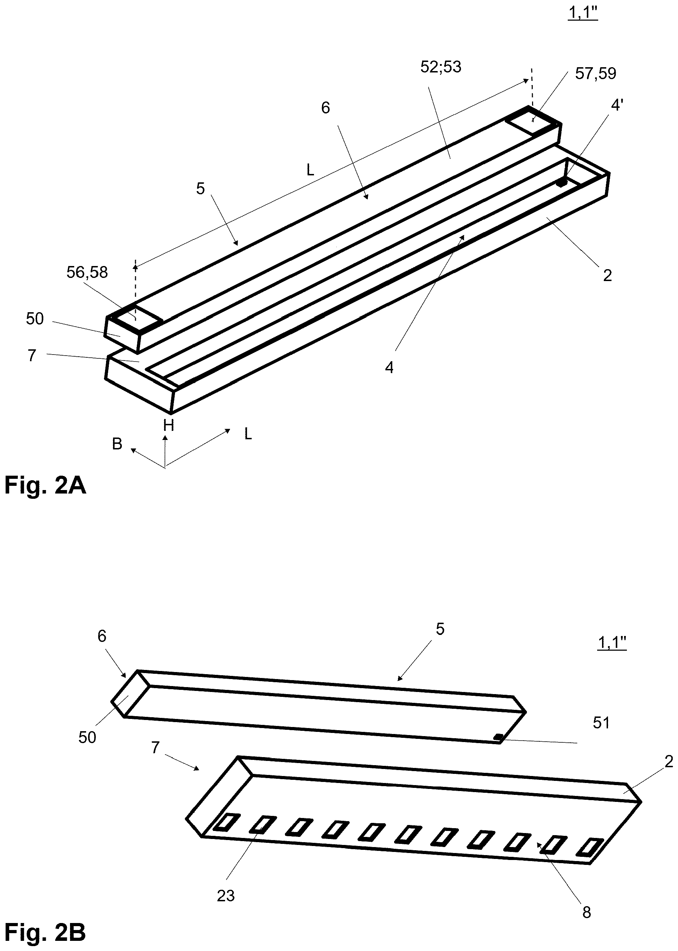

Das Desinfektionsmoduls 5 weist ein eigenes Gehäuse 50 auf und bildet damit eine von den anderen Komponenten der Hygieneleuchte eigenständige Einheit aus. Das Desinfektionsmoduls 5 kann über geeignete Mittel, zum Beispiel magnetisch, über Klippverbindungen oder Schnellspanner lösbar in dem Einbauschacht 4 fixiert werden. Das Desinfektionsmoduls 5 besitzt eine u.a. in Fig. 2B ersichtliche Schnellkupplung 51 zur kombinierten Übertragung von elektrischer Leistung und Steuersignalen. Im Einbauschacht 4 ist hierfür eine in Fig. 2A ersichtliche komplementäre Schnellkupplung 4' vorgesehen, so dass das Desinfektionsmodul 5 über die Schnellkupplung 4' mit Energie und Informationen versorgt werden kann.

Die Schnellkupplung 4' kann auch dazu eingerichtet sein, Zustandsinformationen über das Desinfektionsmodul 5 zur Verfügung zu stellen. Hierzu kann geeignete Auswertungssensorik und -software in dem Desinfektionsmodul verbaut sein. Die Auswertungssensorik und - software kann jedoch auch im Korpus 2 der Hygieneleuchte 1 angeordnet sein.

Bei dem Desinfektionsmodul 5 kann es sich beispielsweise um das in den Figuren 3 bis 4A näher erläuterte Desinfektionsmodul handeln.

Die Oberseite 52 des Gehäuses 50 ist gleichzeitig als Wartungsklappe 53 ausgebildet, so dass ein direkter Zugang zum Inneren des Desinfektionsmoduls 5, insbesondere zu Reinigungs- oder Wartungszwecken, jederzeit und in einigen Ausführungsformen werkzeuglos möglich ist.

In der Oberseite sind zumindest ein Einlass 56 und einen Auslass 57 angeordnet, wobei dem Einlass 56 als auch dem Auslass 57 jeweils ein Gebläse 58, 59 zugeordnet ist, welche für einen erzwungenen Luftstrom durch einen Strömungskanal des Desinfektionsmoduls 5 sorgen. Im Inneren des Desinfektionsmoduls ist dabei eine z.B. in Fig. 3 gezeigte UV-Leuchteinheit 55 angeordnet, über welche vorbeiströmende Luft bestrahlt und damit Krankheitserreger abgetötet werden können.

Der Einbauschacht 4 ist dabei auf der rückseitigen Außenseite 7 der Säule 25 angeordnet, sodass die Luftseite 6, d.h. die Seite, auf welcher der Einlass 56 und der Auslass 57 angeordnet sind, von einem Nutzer der Hygieneleuchte weggerichtet ist. Der Einbauschacht 4 könnte jedoch auch an einer anderen Außenseite der Hygieneleuchte 1 angeordnet sein.

Figuren 2A und 2B zeigen eine Hygieneleuchte 1 in Gestalt einer Pendelleuchte 1" in zwei perspektivischen Ansichten. Die Pendelleuchte 1" besteht aus einem im Wesentlichen quaderförmigen Korpus 2, der analog dem vorhergehenden Ausführungsbeispiel einen Einbauschacht 4 für das Desinfektionsmoduls 5 besitzt.

Im Gegensatz zum vorherigen Ausführungsbeispiel ist die Lichteinheit 3, hier als Aneinanderreihung einer Vielzahl von einzelnen LED-Modulen 23, nicht senkrecht zum Desinfektionsmodul 5, sondern parallel dazu angeordnet (übereinstimmende Längsachsen in Längsrichtung L), wobei die Lichteinheit 3 und das Desinfektionsmodul 5 auf gegenüberliegenden Seiten der Hygieneleuchte 1 angeordnet sind. In anderen Worten weisen die Luftseite 6 sowie die Leuchtseite bzw. Lichtseite 8 in entgegengesetzter Richtung.

Die Pendelleuchte kann beispielsweise über eine Aufhängung 26 oder eine andere geeignete Vorrichtung über einem Arbeitsplatz befestigt werden, sodass der Arbeitsplatz über die Lichteinheit 3 ausreichend beleuchtet wird und gleichzeitig die Luft am Arbeitsplatz durch die erzwungene Luftkonvektion im Desinfektionsmodul 5 kontinuierlich ausgetauscht und aufbereitet wird.

Auch hier können der Einbauschacht bzw. das Desinfektionsmodul auch an bzw. in einer anderen als der hier gewählten von außen zugänglichen Außenseite 7 angeordnet sein.

In den Figuren 3, 4 sowie 3A und 4A ist eine weitere Ausführungsform einer als Pendelleuchte 1" ausgeführten Hygieneleuchte 1 dargestellt, wobei - wie in allen gezeigten Ausführungsbeispielen - gleiche Bauteile oder Funktionen mit gleichen oder vergleichbaren Bezugszeichen versehen sind. So kann die zum ersten Ausführungsbeispiel der Fig. 1 erläuterte Schnellkupplung 4' gleich oder ähnlich auch in den anderen Ausführungsbeispielen vorhanden sein oder zumindest ergänzt werden.

Die Hygieneleuchte 1 besteht dabei aus einem aus gebogenen Blech oder Aluminiumprofil hergestellten Korpus 2, in welchem unter anderem die Lichteinheit 3 sowie das Desinfektionsmoduls 5 verbaut sind. Die Lichteinheit ist dabei aus einer Kette einzelner LED-Module aufgebaut die gemeinsam miteinander verschaltet und über eine Ansteuerung 10 gemeinsam angesteuert werden. Die Leuchteinheit 3 kann über einen Schalter 28 an- und ausgeschaltet werden. Eine dimmbare Ausführungsform, z.B. mit Taster, Drehschalter oder Fernsteuerung mit oder ohne Anbindung an das Gebäudemanagementsystem ist ebenfalls möglich.

Wie in den vorhergehenden Ausführungsbeispielen, handelt es sich bei dem Desinfektionsmodul 5 um ein eigenständiges Bauteil mit einem separaten Gehäuse 50. Das Gehäuse 50 besteht dabei aus einem im Querschnitt U-förmig ausgebildeten Tunnel, vgl. insb. die Schnittansichten der Fig. 3A und 4A, und auf seiner Oberseite 52 mit einem eine als Deckel ausgeführten Wartungsklappe 53 verschlossen. Die Wartungsklappe 53 ist hierüber Schrauben lösbar mit dem übrigen Gehäuse 50 verbunden. Das Gehäuse 50 bildet damit einen im Querschnitt rechteckigen und luft- bzw. druckdichten Strömungskanal 54, vgl. Dichtung 60. Die Strömungsrichtung S im Strömungskanal 54 ist dabei durch die in den Figuren 3 bis 4A ersichtlichen Well-Pfeile angedeutet und richtet sich in Blattebene gesehen also von links nach rechts (Figuren 3 und 3A) bzw. in das Blatt hinein (Figuren 4 und 4A). Das Gehäuse 50 des Desinfektionsmoduls 5 kann hier über eine Vielzahl von Schrauben lösbar mit dem Korpus 2 der Hygieneleuchte 1 befestigt sein.

Der Strömungskanal 54 ist über (in Längsrichtung L gesehen) den am Anfang des Gehäuses befindlichen Einlass 56 sowie den am Ende 57 befindlichen Auslass, welche hier als Gitterschlitze in der Wartungsklappe 53 ausgebildet sind, atmosphärisch verbunden. Unterhalb der Gitterschlitze befindet sich jeweils ein liegend angeordnetes und als Axiallüfter ausgebildete Gebläse 58, 59 mit vertikal angeordneten Rotationsachsen. Über die Gebläse 58, 59 kann eine Luftumwälzung durch den Strömungskanal 54 erzwungen werden. Ein- und Auslass sind über Lichtfallen 61, 62 vor Austritt von UV-Strahlung geschützt. Im Strömungskanal 54 können vorteilhaft auch Filter, insbesondere am Anfang des Strömungskanals S positioniert sein (Filter 63, s. Fig. 4), um eine Verschmutzung des Strömungskanals und insbesondere einer darin angeordneten UV-Leuchteinheit 55 zu vermeiden oder zumindest zu verringern. Für eine gleichbleibende Desinfektionsleistung des Desinfektionsmoduls 5 müssen Filter regelmäßig ausgetauscht oder gereinigt werden.

Im Strömungskanal ist eine hier als UV-Röhre ausgebildete UV-Leuchteinheit 55 angeordnet, welche sich im Wesentlichen über die gesamte Länge des Strömungskanals erstreckt. Die UV-Röhre ist dabei im Strömungskanal nicht mittig oder zentrisch, sondern seitlich angeordnet. Dies verbessert die Zugänglichkeit für Austauscharbeiten der UV-Leuchteinheit 55. Die UV-Leuchteinheit 55 könnte jedoch auch zentrisch angeordnet sein. Durch die UV-Bestrahlung kann durch den Strömungskanal 54 strömende Luft von Viren und Keimen befreit bzw. deren Umfang zumindest erheblich reduziert werden.

An den Seitenwänden des Strömungskanals S sind zusätzlich konkav geformte Reflektorbleche 64, 65 angeordnet, die von der UV-Leuchteinheit emittiertes Strahlung wieder in die Mitte des Strömungskanals zurückwerfen. Die Reflektorbleche 64 haben den Vorteil, dass der Wirkungsgrad der UV-Lichteinheit 55 erhöht und die Verlustleistung (Umwandlung der Strahlung in Wärme) reduziert werden kann. Die Reflektorbleche 64 haben dabei vorzugsweise einen Reflexionsgrad von mehr als 75%, mehr als 85% oder mehr als 95% für die von der UV-Leuchteinheit 55 emittierte Strahlung.

Zur Ansteuerung der Gebläse 58, 59, der UV-Leuchteinheit 55 und der Lichteinheit 3 sind über der Lichteinheit 3 im Korpus 2 der Hygieneleuchte 1 entsprechende Ansteuerungseinheiten 10, 11 und 12 angeordnet. Gebläse 58, 59 und UV-Leuchteinheit 55 sind parallelgeschaltet und können dabei über einen gemeinsamen Schalter 27 an- bzw. ausgeschaltet werden. Ein weiterer Schalter 28 ist für das An- und Ausschalten der Lichteinheit 23 vorgesehen.

In weiteren Abwandlungen der Hygieneleuchte 1 können die Ansteuerungen 10, 11 und 12 auch gemeinsam in einem (nicht dargestellten) Steuermodul integriert sein, wobei das Steuermodul eine Schnittstelle an ein Gebäudemanagementsystem bzw. ein Gebäude-Bus-System aufweisen kann. Hierdurch ist beispielsweise eine ferngesteuerte Schaltung der einzelnen Komponenten der Hygieneleuchte möglich.

In anderen vorteilhaften Abwandlungen können Zeituhren, Volumenmesser oder andere zweckmäßige Sensoren im Desinfektionsmodul oder in anderen Teilen der Hygieneleuchte zur Zustandsermittlung der Komponenten der Hygieneleuchte verbaut sein. Das Steuermodul kann die von dem oder den Sensoren zur Verfügung gestellten Daten auswerten und darauf basierend Handlungsempfehlungen an den Betreiber oder Operateur der Hygieneleuchte ausgeben, beispielsweise als Ferndiagnose via LAN oder WAN. Die Hygieneleuchte kann hierzu geeignete Kommunikationsschnittstellen aufweisen.

Die Lichteinheit 3 kann in Abwandlungen der Ausführungsformen vorzugsweise als baulich selbstständige Einheit ausgebildet sein, welche analog dem Desinfektionsmodul zu einfachen Wartungszwecken demontierbar ausgebildet ist. Die Lichteinheit 3 kann jedoch auch aus einzelnen baulich getrennten Lichtmodulen zusammengesetzt sein, welche z.B. getrennt in den Korpus 2 der Hygieneleuchte 1 eingesetzt sind bzw. werden. Auch eine Linear-LED mit z.B. prismatischer oder opaler Abdeckung als Lichteinheit 3 ist denkbar.

Die erfindungsgemäße Hygieneleuchte mit einem eigenständigen Desinfektionsmodul erlaubt eine schnelle und einfache Wartung des Desinfektionsmoduls ohne längere Ausfallzeiten der Hygieneleuchte. Insbesondere können reinigungsbedürftige, wartungsbedürfte oder defekte Desinfektionsmodule innert weniger Sekunden ausgetauscht und durch ein funktionsfähiges Desinfektionsmodul ersetzt werden.

Die Ausführung des Desinfektionsmoduls als eigenständige Baueinheit erlaubt darüber hinaus die Bereitstellung verschiedener Desinfektionsmodule mit unterschiedlicher Leistung, die in baugleiche Einbauschächte eingesetzt werden können, so dass ein Anwender flexibel auf veränderte Bedarfe bezüglich einer Luftaufbereitungs- bzw. Luftreinigungsleistung durch Austausch eines Desinfektionsmoduls mit höherer oder niedriger Leistung reagieren kann.

Alle Desinfektionsmodule können dabei als plug-and-play-Systeme ausgebildet sein, die beim Einbau in die entsprechenden Hygieneleuchten von Steuermodulen oder -software automatisch erkannt und angesteuert werden.

### Bezugszeichen

- L: Länge (Desinfektionsmodul)
- S: Strömungsrichtung

- 1: Hygieneleuchte
- 1": Pendelleuchte
- 1': Stehlampe

- 2: Corpus (Lampe)
- 3: Lichteinheit
- 4: Einbauschacht
- 4': Schnellkupplung, elektrisch

- 5: Desinfektionsmodul
- 6: Luftseite (gegenüberliegend oder senkrecht zueinander ausgerichtet)
- 7: Außenseite
- 8: Lichtseite

- 10: Ansteuerung Lichteinheit
- 11: Ansteuerung UV-Leuchteinheit
- 12: Ansteuerung Gebläseeinheit

- 21: Oberseite (Lampe)
- 22: Aufhängung
- 23: LED-Modul
- 24: Standfuß (Stehlampe)
- 25: Säule (Stehlampe)
- 26: Aufhängung (Pendelleuchte)
- 27: Schalter Desinfektionsmodul
- 28: Schalter Lichteinheit

- 50: Gehäuse (Desinfektionsmodul)
- 51: Schnellkupplung, elektrisch
- 52: Oberseite (Desinfektionsmodul)
- 53: Wartungsklappe
- 54: Strömungskanal
- 55: UV-Leuchteinheit
- 56: Lufteinlass
- 57: Luftauslass
- 58: Gebläse
- 59: Gebläse
- 60: Dichtung
- 61: Lichtfalle
- 62: Lichtfalle
- 63: Filter
- 64: Reflektor
- 65: Reflektor

## Patentansprüche

1. Hygieneleuchte (1) umfassend einen Korpus (2), in dem eine Lichteinheit (3) sowie ein Desinfektionsmodul (5) angeordnet sind, wobei das Desinfektionsmodul (5) ein Gehäuse (50) mit einem Einlass (56), einem Auslass (57) und einem sich dazwischen erstreckenden Strömungskanal (54) sowie eine im Strömungskanal (54) angeordnete UV-Leuchteinheit (55) umfasst und wobei in dem Korpus (2) ein zu dessen Außenseite (7) hin offener Einbauschacht (4) für das Desinfektionsmodul (5) angeordnet ist, in welchem das Desinfektionsmodul (5) mit seinem Gehäuse (50) als gesamte Einheit demontierbar aufgenommen ist, **dadurch gekennzeichnet, dass** in dem Desinfektionsmodul (5) am Einlass und/oder am Auslass (56 bzw. 57) des Gehäuses (50) ein als Axiallüfter ausgebildetes Gebläse (58, 59) senkrecht zu einer Strömungsrichtung (S) angeordnet ist.

2. Hygieneleuchte (1) nach Anspruch 1, **dadurch gekennzeichnet, dass** das Desinfektionsmodul (5) eine elektrische und/oder mechanische Schnellkupplung (51) zur Verbindung mit einem komplementären Gegenstück (4') im Einbauschacht (4) aufweist.

3. Hygieneleuchte (1) nach einem der vorhergehenden Ansprüche, **dadurch gekennzeichnet, dass** das der Einbauschacht (4) als einseitig offene Vertiefung ausgebildet ist, in welchem das Desinfektionsmodul (5) vorzugsweise bündig abschließend aufgenommen ist.

4. Hygieneleuchte (1) nach einem der vorhergehenden Ansprüche, **dadurch gekennzeichnet, dass** das Desinfektionsmodul (5) eine Wartungsklappe (53) an seiner Oberseite (52) aufweist, über welches Komponenten des Desinfektionsmoduls (5) ohne Ausbau des Desinfektionsmoduls (5) unmittelbar zu Wartungszwecken zugänglich sind.

5. Hygieneleuchte (1) nach einem der vorhergehenden Ansprüche, **dadurch gekennzeichnet, dass** im Strömungskanal (54) des Desinfektionsmoduls (5) eine nach dem Einlass (57) angeordnete Lichtfalle (61) und/oder eine vor dem Auslass (59) angeordnete Lichtfalle (62) angeordnet ist/sind.

6. Hygieneleuchte (1) nach einem der vorhergehenden Ansprüche, **dadurch gekennzeichnet, dass** im Strömungskanal (54) wenigstens ein sich vorzugsweise über die gesamte Längserstreckung (L) des Strömungskanals (54) erstreckender Reflektor (64, 65) ausgebildet ist.

7. Hygieneleuchte (1) nach einem der vorhergehenden Ansprüche, **dadurch gekennzeichnet, dass** die Lichteinheit (3) und das Desinfektionsmodul (5) parallel, insbesondere mit voneinander wegweisender Lüftungs- und Lichtseite (8 bzw. 6), oder senkrecht zueinander angeordnet sind.

8. Hygieneleuchte (1) nach einem der vorhergehenden Ansprüche, **dadurch gekennzeichnet, dass** die Hygieneleuchte (1) als Stehlampe (1') mit einem Standfuß (24) und einer Säule (25) ausgebildet ist, wobei der Einbauschacht (4) für das Desinfektionsmodul (5) in der Säule (25) der Stehlampe (1') angeordnet ist.

9. Hygieneleuchte (1) nach einem der Ansprüche 1 bis 8, **dadurch gekennzeichnet, dass** die UV-Leuchteinheit (55) ein gemeinsames Steuermodul oder jeweils ein separates Steuermodul zur mittel- oder unmittelbaren Ansteuerung von UV-Leuchteinheit (55), des oder der Gebläse (58, 59) und/oder der Lichteinheit (3) aufweist.

10. Hygieneleuchte (1) nach Anspruch 9, **dadurch gekennzeichnet, dass** das Steuermodul (13) eine Schnittstelle zur Verbindung mit einem Schalter (27) und/oder zur Anbindung an ein Gebäudemanagement-System aufweist.

11. Hygieneleuchte (1) nach einem der Ansprüche 9 oder 10, **dadurch gekennzeichnet, dass** das Steuermodul die Leistung der UV-Leuchteinheit (55) und/oder des oder der Gebläse (58,59) abhängig oder unabhängig voneinander regelt.

12. Hygieneleuchte (1) nach einem der vorhergehenden Ansprüche, **dadurch gekennzeichnet, dass** die Hygieneleuchte (1) einen Bewegungsmelder und/oder eine Zeitschaltuhr umfasst, welcher das Lichtmodul (3) und/oder das Desinfektionsmodul (5) automatisch steuert.

13. Hygieneleuchte (1) nach einem der vorhergehenden Ansprüche, **dadurch gekennzeichnet, dass** in dem Desinfektionsmodul (5) ein Sensor zur Erfassung des Betriebszustands angeordnet ist.

14. Hygieneleuchte (1) nach einem der vorhergehenden Ansprüche, **dadurch gekennzeichnet, dass** die Hygieneleuchte (1) eine Kommunikationsschnittstelle aufweist, über welche Zustandsinformationen zur Hygieneleuchte (1) zur Verfügung gestellt werden können und/oder über welche die Leistung der Leuchteinheit (3) und/oder des Desinfektionsmoduls (5), insbesondere die Leistung eines Gebläses (56, 57) und/oder der Leistung der UV-Leuchteinheit (55), regelbar ist.

## Claims

1. Hygiene lamp (1) comprising a body (2) in which a light unit (3) and a disinfection module (5) are arranged,
wherein the disinfection module (5) comprises a housing (50) with an inlet (56), an outlet (57) and a flow channel (54) extending therebetween
and a UV light unit (55) arranged in the flow channel (54),
wherein an installation slot (4) for the disinfection module (5), which is open towards the outside (7) of the body (2), is arranged in the body (2), in which the disinfection module (5) with its housing (50) is accommodated in such a way that it can be removed as a complete unit, **characterised in that** in the disinfection module (5) at the inlet and/or at the outlet (56 or 57) of the housing (50), an air blower (58, 59) designed as an axial fan is arranged perpendicular to a flow direction (S).

2. Hygiene lamp (1) according to claim 1, **characterised in that** the disinfection module (5) has an electrical and/or mechanical quick coupling (51) for connection to a complementary counterpart (4') in the installation slot (4).

3. Hygiene lamp (1) according to any one of the preceding claims, **characterised in that** the installation slot (4) is designed as a recess which is open on one side and in which the disinfection module (5) is preferably accommodated in a flush manner.

4. Hygiene lamp (1) according to one of the preceding claims, **characterised in that** the disinfection module (5) has a maintenance flap (53) on its upper side (52), via which components of the disinfection module (5) are directly accessible for maintenance purposes without removing the disinfection module (5).

5. Hygiene lamp (1) according to any one of the preceding claims, **characterised in that** a light trap (61) arranged downstream of the inlet (57) and/or a light trap (62) arranged upstream of the outlet (59) is/are arranged in the flow channel (54) of the disinfection module (5).

6. Hygiene lamp (1) according to one of the preceding claims, **characterised in that** at least one reflector (64, 65) extending preferably over the entire longitudinal extent (L) of the flow channel (54) is formed in the flow channel (54).

7. Hygiene lamp (1) according to any one of the preceding claims, **characterised in that** the light unit (3) and the disinfection module (5) are arranged parallel to each other, in particular with the ventilation and light side (8 and 6 respectively) facing away from each other, or perpendicular to each other.

8. Hygiene lamp (1) according to any one of the preceding claims, **characterised in that** the hygiene lamp (1) is designed as a floor lamp (1') with a pedestal (24) and a column (25), the installation slot (4) for the disinfection module (5) being arranged in the column (25) of the floor lamp (1').

9. Hygiene lamp (1) according to any one of the claims 1 to 8, **characterised in that** the UV lamp unit (55) has either a common control module or a separate control module for indirect or direct control of the UV lamp unit (55), of the blower(s) (58, 59) and/or of the light unit (3), respectively.

10. Hygiene light (1) according to claim 9, **characterised in that the** control module (13) has an interface for connection to a switch (27) and/or for connection to a building management system.

11. Hygiene lamp (1) according to any one of claims 9 or 10, **characterised in that** the control module (13) controls the power of the UV lamp unit (55) and/or the blower(s) (58,59) dependent on or independently of each other.

12. Hygiene light (1) according to any one of the preceding claims, **characterised in that** the hygiene light (1) comprises a motion detector and/or a timer which automatically controls the light module (3) and/or the disinfection module (5).

13. Hygiene lamp (1) according to any one of the preceding claims, **characterised in that** a sensor for detecting the operating state is arranged in the disinfection module (5).

14. Hygiene lamp (1) according to any one of the preceding claims, **characterised in that** the hygiene lamp (1) has a communication interface via which status information of the hygiene lamp (1) can be made available and/or via which the power of the lamp unit (3) and/or of the disinfection module (5), in particular the power of a blower (56, 57) and/or the power of the UV lamp unit (55), can be regulated.

## Revendications

1. Lampe à usage hygiénique (1) comprenant un corps (2), dans lequel sont disposés une unité lumineuse (3) ainsi qu'un module de désinfection (5), dans laquelle le module de désinfection (5) comprend un boîtier (50) avec une entrée (56), une sortie (57) et un canal d'écoulement (54) s'étendant entre celles-ci ainsi qu'une unité d'éclairage UV (55) disposée dans le canal d'écoulement (54) et dans laquelle dans le corps (2) est disposée une baie d'installation (4) ouverte en direction de la face extérieure de celui-ci (7) pour le module de désinfection (5), dans lequel le module de désinfection (5) avec son boîtier (50) est logé de manière démontable en tant qu'unité d'ensemble, **caractérisée en ce qu'**une soufflante (58, 59) réalisée sous la forme d'un ventilateur axial est disposée perpendiculairement à une direction d'écoulement (S) dans le module de désinfection (5) à l'entrée et/ou à la sortie (56 ou 57) du boîtier (50).

2. Lampe à usage hygiénique (1) selon la revendication 1, **caractérisée en ce que** le module de désinfection (5) présente un accouplement rapide électrique et/ou mécanique (51) pour la liaison à une pièce homologue complémentaire (4') dans la baie d'installation (4).

3. Lampe à usage hygiénique (1) selon l'une quelconque des revendications précédentes, **caractérisée en ce que** la baie d'installation (4) est réalisée sous la forme d'un évidement ouvert d'un côté, dans lequel le module de désinfection (5) est logé de préférence de manière à terminer en affleurement.

4. Lampe à usage hygiénique (1) selon l'une quelconque des revendications précédentes, **caractérisée en ce que** le module de désinfection (5) présente un clapet de maintenance (53) sur sa face supérieure (52), par l'intermédiaire duquel les composants du module de désinfection (5) sont directement accessibles à des fins de maintenance sans démontage du module de désinfection (5) .

5. Lampe à usage hygiénique (1) selon l'une quelconque des revendications précédentes, **caractérisée en ce qu'**une trappe lumineuse (61) disposée après l'entrée (57) et/ou une trappe lumineuse (62) disposée avant la sortie (59) est/sont disposées dans le canal d'écoulement (54) du module de désinfection (5).

6. Lampe à usage hygiénique (1) selon l'une quelconque des revendications précédentes, **caractérisée en ce qu'**au moins un réflecteur (64, 65) s'étendant de préférence sur toute l'étendue longitudinale (L) du canal d'écoulement (54) est réalisé dans le canal d'écoulement (54).

7. Lampe à usage hygiénique (1) selon l'une quelconque des revendications précédentes, **caractérisée en ce que** l'unité lumineuse (3) et le module de désinfection (5) sont disposés parallèlement, en particulier avec une face de ventilation et lumineuse (8 ou 6) orientée dans la direction opposée l'une à l'autre, ou perpendiculairement l'une à l'autre.

8. Lampe à usage hygiénique (1) selon l'une quelconque des revendications précédentes, **caractérisée en ce que** la lampe à usage hygiénique (1) est réalisée sous la forme d'un lampadaire (1') avec un pied (24) et une colonne (25), dans laquelle la baie d'installation (4) pour le module de désinfection (5) est disposée dans la colonne (25) du lampadaire (1').

9. Lampe à usage hygiénique (1) selon l'une quelconque des revendications 1 à 8, **caractérisée en ce que** l'unité d'éclairage UV (55) présente un module de commande commun ou respectivement un module de commande séparé pour la commande indirecte ou directe de l'unité d'éclairage UV (55), de la ou des soufflantes (58, 59) et/ou de l'unité lumineuse (3).

10. Lampe à usage hygiénique (1) selon la revendication 9, **caractérisée en ce que** le module de commande (13) présente une interface pour la liaison à un commutateur (27) et/ou pour le raccordement à un système de gestion de bâtiment.

11. Lampe à usage hygiénique (1) selon l'une quelconque des revendications 9 ou 10, **caractérisée en ce que** le module de commande régule la puissance de l'unité d'éclairage UV (55) et/ou de la ou des soufflantes (58, 59) en fonction ou indépendamment les unes des autres.

12. Lampe à usage hygiénique (1) selon l'une quelconque du revendications précédentes, **caractérisée en ce que** la lampe à usage hygiénique (1) comprend un détecteur de mouvement et/ou un temporisateur, lequel commande automatiquement le module lumineux (3) et/ou le module de désinfection (5).

13. Lampe à usage hygiénique (1) selon l'une quelconque du revendications précédentes, **caractérisée en ce qu'**un capteur pour la détection de l'état de fonctionnement est disposé dans le module de désinfection (5).

14. Lampe à usage hygiénique (1) selon l'une quelconque du revendications précédentes, **caractérisée en ce que** la lampe à usage hygiénique (1) présente une interface de communication, par l'intermédiaire de laquelle des informations d'état pour la lampe à usage hygiénique (1) peuvent être fournies et/ou par l'intermédiaire de laquelle la puissance de l'unité d'éclairage (3) et/ou du module de désinfection (5), en particulier la puissance d'une soufflante (56, 57) et/ou la puissance de l'unité d'éclairage UV (55), peut être régulée.
